# EUROPEAN PATENT APPLICATION

(11) **EP 4 834 381 A1**
(43) Date of publication of application: **04.11.2026**
(21) Application number: 25173768.0
(22) Date of filing: 30.04.2025
(51) Int. Cl.: A61M 1/00

(54) **PUMPING DEVICE FOR REDUCED PRESSURE THERAPY**

(71) Applicant: MEDAXIS AG, 6340 Baar (CH)
(72) Inventor: Geiser, Nico, 5102 Rupperswil (CH); Bernhard, Jérôme, 8003 Zürich (CH); Larsson, Michael, 6302 Zug (CH); Christen, Lukas, 6004 Luzem (CH); Schneebeli, Lucian, 8047 Zurich (CH); Napoletano, Daniel, 8455 Rüdlingen (CH)
(74) Representative: Grünecker Patent- und Rechtsanwälte PartG mbB

(57) **Abstract**

The present invention relates to a pumping device and a computerized method for management of a reduced pressure therapy. The pumping device (2) for reduced pressure therapy of a wound (24) covered by a dressing (18) comprises a housing (10), a pump (30) comprising a suction port (32), a controller (28) configured to selectively drive the pump, a battery compartment (38) adapted to receive at least one battery (42) for energizing the pump, a pressure interface (14) in fluid communication with said suction port (32), which pressure interface (14) is accessible from outside for being connected to a fluid connection (16) leading to the dressing (18), at least one of a pressure relief valve (34) and a pressure sensor (36) adapted to provide a signal indicative of a suction pressure at the suction port (32). The present invention aims to provide a pumping device which is inexpensive to manufacture and/or provides low energy consumption and/or low noise emission. Furthermore, the pumping device shall provide improved patient monitoring and patient compliance. According to one aspect of the invention a manifold body (60) is provided for fluidically connecting the suction port (32), the pressure interface (14) and the at least one of said pressure relief valve (34) and said pressure sensor (36).

## Description

The present invention relates to a pumping device and a computerized method for management of a reduced pressure therapy.

The benefits of reduced pressure therapy are well accepted. Formally, reduced pressure therapy was carried out in a hospital in which the patient was covered by a wound dressing, which wound dressing was essentially airtight and provided with a fluid connection. That fluid connection in form of a hose led to a stationary pump which was energized and controlled to apply a negative pressure/suction within the wound to e.g., foster microcirculation in the wound ground, and allow the dressing to rest on the wound for longer time as the exudate was drawn from the dressing and into a canister assigned to the pump.

Those stationary solutions are nowadays at least in part replaced by mobile pumping devices which are rather small and are combined by dressings suitable to store the exudate expected to be released in the wound ground. In other words, the small pumping device does not require a canister.

Such pumping device is e.g., known from US 10 828 403 B2. The pumping device for such mobile use is usually as a disposable item designed for single use only usually over a limited period of time. Power supply of the pumping device is provided by batteries which usually can be taken out of a battery compartment of the pumping device and thus be disposed separately from the pumping device. The pumping device itself will be regarded as special waste as it may have come in contact with contaminated body fluid and thus must be disposed like any other surgical disposable.

Due to the nature of the pumping device, the same must be small and inexpensive to manufacture. As the pumping device is permanently worn by the user specific attention is required for reducing the noise made by the pumping device during use.

The present invention aims to provide a pumping device which is inexpensive to manufacture and/or provides low energy consumption and/or low noise emission. Furthermore, the pumping device shall provide improved patient monitoring and patient compliance.

As a solution to this object the present invention provides a pumping device comprising a housing, a pump comprising a suction port, a controller configured to selectively drive the pump, a battery compartment adapted to receive at least one battery for energizing the pump and a pressure interface in fluid communication with said with said suction port, which pressure interface is accessible from outside to a fluid connection leading to the dressing. Furthermore, there is provided at least one of a pressure relief valve for relieving the wound from excessive negative pressure provided by the pumping device and/or a pressure sensor for monitoring the pressure effective on the wound. The pressure relief valve is usually a passive valve which vents the pumping device in any event in case the pump produces an improper high suction, which may impair the healing process and/or cause pain on the wound. The pressure sensor is adapted to provide a sensor signal indicative of a suction pressure at the suction port. This indication must not reflect exactly the pressure existing above the wound ground and thus within the dressing.

The inventive pumping device has a manifold body for fluidly connecting the suction port, the pressure interface and at least one of the pressure relief valve and the pressure sensor. Usually the manifold body provides a plug receptacle for plug connecting the suction port, the pressure interface and the at least one of the pressure relief valve and the pressure sensor. The manifold body may provide three or up to five or even more plug receptacles and thus interfaces to receive components of the pumping device in a sealing manner. For this the manifold body preferably is made of an elastic material which sealing surrounds the suction port of the pump. The elastic material preferably is a plastic material.

The suction port of the pump may be plug connected with the manifold body or the entire or at least part of the pump may be plug connected with the manifold body. The manifold body, furthermore, has a plug connection for a pressure interface body providing the pressure interface. This pressure interface body may have a male connection section adapted to be inserted into the receptacle of the manifold body and a Luer lock end which surrounds the suction port and is adapted to be connected with a female Luer lock provided on a distal end of the fluid connection. The manifold body furthermore has a plug receptacle for a pressure relief valve body of the pressure valve and/or a pressure sensor body of the pressure sensor.

Moreover the manifold body may provide a plug connection for a check valve which check valve usually is received within the manifold body and arranged between the suction port of the pump and the pressure interface body both being received at least in part within the manifold body. The check valve will improve efficiency of the pump and thus will help to maintain a higher suction pressure/negative pressure at the suction port and thus within the dressing.

The manifold body usually has four preferably up to five or even more plug receptacles. Usually the manifold body has at least the receptacles for the pressure interface body, the suction port, the pressure sensor and the pressure relief valve provided to by all means avoid excessive suction pressure effective in the wound which may cause pain. The pressure relief valve will usually avoid any pressure below minus 200 mmHg.

The pressure sensor will provide feedback information for the controller. The pressure sensor is usually provided as a surface mounted device of a PCB of the controller. In the same way the pump may be directly connected to the PCB. In addition, any switch to switch the pumping device on and off may be surface mounted on the PCB. Preferably, any electric and electronic device of the pumping device is surface mounted on the PCB. The pumping device usually has only one PCB, said PCB also electrically connects and holds at least one light source, preferably an LED or LED arrangement providing optical indication of at least one operational parameter of the pumping device.

As mentioned above the manifold body is preferably made of an elastic or elastomeric material preferably in order to reduce the overall parts and thus simplify assembly of the pumping device. The manifold body is provided adapted to at least in part receive the pump for sound proving of the pump. Thus, the sleeve surrounds entirely or at least partially the pump and reduces the emission of noise by the pump. This includes the option to not only surround an arrangement of the pump leading to a directed airflow but also to surround the motor effecting movement within respective arrangement. Preferably, the motor is also circumferentially surrounded by the sleeve. The sleeve usually has only one open end which is level or projects a free end of the motor of the pump.

The pump, in addition, only has one port, which means that the pump does not have a discrete vent opening to release the overpressure of the pump. Instead, the pump is provided with a pump housing allowing to leak air and thus vent the pump at reduced noise emission.

According to a further aspect of the present invention the pumping device with or without the above mentioned manifold body has a housing formed of at least by two housing shells. The housing shells are sealingly connected with each other e.g. by gluing, and/or welding to hermetically seal a housing compartment defined within the housing. The housing compartment is in communication with ambient only via a hydrophobic filter which is permeable to gas and not permeable to water. The aforementioned pressure interface body is usually received between the housing shell elements in a sealing manner. The quality of the sealing is such that the pumping device fulfills standard IP55. Thus, the user of the pumping device may shower with the pumping device. Prior arc pumping devices must be disconnected from the fluid connection to the wound dressing before showering. Thus, the use will have to shower with the wound dressing in place but not the pumping device. In fact, the quality of sealingly connecting the housing shell elements preferably meets the requirement according to IP67 and thus will allow the user to swim or bath with the pumping device in place.

The hydrophobic filter will be combined with at least one preferably plural openings within at least one of the two housing shell elements. In order to avoid blocking of the hydrophobic filter the same is preferably provided underneath an eyelet and/or a carrying strap which is provided by the housing e.g., to allow carrying or otherwise suspending the housing via the carrying strap. The respective carrying strap is guided through said eyelet. For the carrying strap, the housing is provided with this eyelet on the outer side thereof. Thus even without a carrying strap in place the walls of the eyelet will prevent any clothing or other obstacle to block the hydrophobic filter.

For the same reason as the eyelet and the carrying strap, the housing preferably is provided with an integrated fastening clip. The fastening clip is integral part of at least one of the housing shell elements. Usually the clip is provided on a side opposite to the main side of the housing being provided with the switch and/or the signaling light sources/LEDs.

According to a further aspect of the present invention the pumping device thereof is provided with a reduced weight of not more than 150 g, preferably not more than 125 g, most preferably not more than 100 g. For this, the energy source of the pumping device is preferably limited by two AAA batteries received within the housing. In order to allow separate disposal of the housing with the components contained therein on one hand and the batteries on the other, the same are usually provided within a separate battery compartment which is accessible from outside or at least can be disassembled with the batteries when disposing the pumping device. The pumping device is provided as a low energy consuming pumping device for which the check valve will reduce suction loss between the pump and the suction port. Moreover signaling lights may only be switched on in case emotion is sensed by an acceleration sensor indicating that the user of the pump is active. For example during night when the user is sleeping the energy consumption is limited to energizing the pumping device. Respective acceleration sensor may not only be operatively connected to the controller of the pumping device to control operation of the pump but may also be connected to a memory of respective controller to store movement data sensed by the acceleration sensor and indicative of the movement of the pumping device. Respective movement is indicative of the activity of the user and thus may be utilized to monitor the patient by reading out respective data from the memory. This feature can be used to check whether the user and thus the patient complies with a specific prescription of the clinical personnel in terms of activity level considered to be beneficial for healing of the wound under negative pressure.

As mentioned before the housing preferably has a battery compartment adapted to receive the at least one battery. In view of the IP55 or IP67 standard, respective battery compartment is releasably and sealingly covered by a battery compartment cover provided above the battery compartment at the end of the AAA in longitudinal direction thereof. i.e. above the +/-pole. Such design will allow provision of a battery compartment cover with limited size which size will facilitate sealing of the battery compartment and thus the housing. For properly sealing, the battery compartment may be secured to the housing by means of a force locked connection and/or a positive connection. For example, a snap connection may sealingly press the battery compartment cover against the remaining housing and thus improve sealing of the battery compartment. The seal may be labyrinth seal per se or in combination with the snap seal, which presses surfaces against each other by a snapping force. The seal may likewise comprise an elastomeric component like a separate sealing ring or a sealing arrangement co-injection molded with the housing and/or the battery compartment cover.

According to a further aspect of the present invention the pumping device has specific control means for which it is not necessary to provide the pumping device with the manifold body of the present invention and/or the features leading to a limited weight of the pumping device. The controller may be operatively connected with the pressure sensor and can evaluate a pressure gradient calculated from pressure values sensed by the pressure sensor during suction priming the dressing for selectively setting a suction yield of the pump. Suction priming the dressing is considered to address the initial phase of operation of the pump, in which the dressing connected to the skin of the user and placed above the wound is set under negative pressure for the first time. The pressure gradient sensed during suction priming will give an indication on a leaking dressing which indication may lead to a signal like an audible or haptic or visual alarm. Such alarm may likewise result from insufficient suction or increasing negative pressure within the dressing sensed by the pressure sensor during full operation of the pumping device and indicative of a fresh leak. The same may be true for an obstruction sensed by the sensor.

Moreover, the pressure gradient during priming may be indicative of the size of the dressing in order to adjust the operation of the pump and in particular the yield of the pump to the different sizes of dressings. The sensed and calculated pressure gradient during priming may be used by the controller to automatically set the yield of the pump. A larger dressing will lead to a higher yield of the pump and thus suction capacity and/or suction flow speed of the pump than a smaller dressing. This measure as well may lead to a low energy consumption when operating the pump at least in connection with a smaller or a medium-sized dressing. The controller may, for example, comprise a lookup table with three or more different categories for sizes of dressings and categorize an existing dressing during the priming process in response to the pressure gradient measured when applying suction priming. For this, the memory may have separate predetermined yield values of the pump for each of the different categories for dressing sizes.

In addition or alternatively, the controller may be adapted to apply an initial suction level and order an operational suction level after a predetermined running time of the pumping device. The operational suction level has a higher negative pressure than the initial suction value. In other words, the initial suction value causes a higher pressure within the wound than the operational suction level and can be used to accustom the user and the wound ground to the existing suction pressure. The predetermined running time is usually between 20 and 30 hours. The initial application of the initial suction value will lower pain at the user's side in comparison to an operation in which the operational suction level is applied from the very beginning of the operation of the pumping device.

For monitoring patient compliance, the controller may have a memory which is configured to store data including operational parameters of the pumping device and signals sensed by at least one of the sensors of the pumping device. Furthermore, the pumping device comprises an interface like NFC, Bluetooth or wireless broadband communication interface like LTE. Respective interfaces may be provided in combination and are usually mounted directly on the PCB of the controller. The pumping device furthermore is adapted to read out the stored data for allowing analysis of operational parameters and/or signals sensed by the sensor to analyze the patient individually or cluster information of plural patients for statistically evaluating pumping performance and/or healing progress for which data may be entered by the physician on healing progress of each individual patient. As mentioned before, such data may be read out wirelessly or via a wire connection. Such data may be read out via a docking station. The docking station may likewise be connected wirelessly for providing data connection with the interface for reading out stored data from the memory or reading out data into the memory. The latter being an option to prolong a predetermined lifetime, which lifetime is usually stored as a threshold value, which threshold value by all means will apply and automatically stop operation of the pump after a predetermined time interval has lapsed after starting movement of the pumping device. The threshold operational time is to safeguard reliable operation of the pump during the prescribed therapy time.

For further reducing the weight and potentially the energy consumption of the pump, the same is provided with a single switch for switching on and off the pump. The switch is provided on the outer side of the housing for activating the pumping device. Even a single switch may allowed to order different operational parameters by different ways of entering signals through the one and only switch. The switch preferably is a sliding switch, which not only activates the pump but also provides indication on the operational status of the pump by the position of the switch. Thus, such pump with a sliding switch may not need to have an active or additional optical indicator on the on/off status of the pump, which reduces energy consumption when operating the pump.

The pumping device, furthermore is combinable preferably with a computer application, which computer application is run on a mobile device for visualizing and/or adjusting at least one operational parameter of the pumping device. The connection between the mobile device and the controller may be by wire or wirelessly. The mobile device may be used to input via an input interface, input information related to the operation of the pumping device for the reduced pressure therapy. The mobile device may likewise provide an output interface for outputting an indication to the user. The app and thus the method of the present invention may process by means of a processing circuitry configured by the computer application/app, the input information obtained via the input interface to obtain an indication on at least one operational parameter and to output via the output interface the indication to the user. In other words, the mobile device can be used to input and output information in connection with the use of the pumping device. The information may relate to operational parameter and/or data sensed by at least one of the sensors.

According to a parallel aspect of the present invention, the same provides a computerized method for reduced pressure therapy management, which may run as an app on a mobile device. In this method, input information obtained via the input interface and pre-stored calendar information is processed to obtain an operational parameter to pause operation of the pumping device and to transmit via a pump interface respective operational parameter to the pumping device. Processing will provide an indication on pausing the operation of the pumping device and output via the output interface the indication to the user that the operation is paused. This includes stopping operation of the pump, but also resuming the operation of the pump triggered by calendar information. For example, the calendar of the mobile device may pause operation of the pump in case a business meeting is scheduled in the calendar for a predetermined period of time in order to avoid operation of the pump, which despite all measures for reducing the noise may lead to acoustic irritation during the meeting.

In addition or alternatively, the application may correlate healing process information with operational or sensed information of the pumping device. The healing progress information may be entered manually as parameter information or be represented by a sequence of photos of the wound showing progress of wound healing. Other parameters for healing of the wound like thrombocyte counts may be entered as healing progress information. Any healing progress information is correlated by artificial intelligence with the input information related to the operation of the pump and/or the input sensor information to evaluate performance of the pump with respect to healing progress. The correlation by artificial intelligence may but must not be performed on the mobile device of the user. Instead, respective correlation may be carried out on a central server receiving information from plural pumping devices e.g. via mobile devices each being connected with one pumping device and with the central server via internet, which eventually will allow optimization of pumping parameters in terms of optimum healing performance.

In addition or alternatively, the application may help the user to improve understanding of operational parameters visualized e.g., by LED and/or sound. For this, the application may be adapted to correlate via a processing circuitry, pre-stored instruction for use and a help request entered by the user via the mobile device to obtain help information. By means of the app, respective help requests will be correlated with the pre-stored information to output the help information to the user. Respective preferred embodiment may help to read out instruction for use of the pumping device by the user.

Finally, the present invention proposes a negative pressure therapy kit. This kit comprises an embodiment of the pumping device of the invention, a dressing, a fluid connection coupleable with the dressing and the pumping device and configured to provide a fluid path of reduced pressure between the dressing and the pumping device. The negative pressure kit is sterile and contained in sterile condition in at least one packaging element. This, the kit can be used on the sterile side of an operation room, in which the wound of the user is covered in a sterile environment.

Further details, advantages or features of the present invention will become apparent from the following description of a specific embodiment in combination with the drawings. In the drawings:
- Fig. 1: is an exploded perspective view of the embodiment of a pumping device of the present invention;
- Fig. 2: is a perspective view of the embodiment with a fluid connection leading to a dressing;
- Fig. 3: is a side view of the pumping device of Fig. 1;
- Fig. 4: is a front view of the pumping device of Figs. 1 and 3;
- Fig. 5: is a side view of a side opposite to the side shown in Fig. 3;
- Fig. 6: is a graph exemplifying different pressure gradients;
- Fig. 7: is scheme showing wireless connections of the pumping device and
- Fig. 8: is a graph showing the effective pressure in the dressing over time during the reduced pressure therapy.

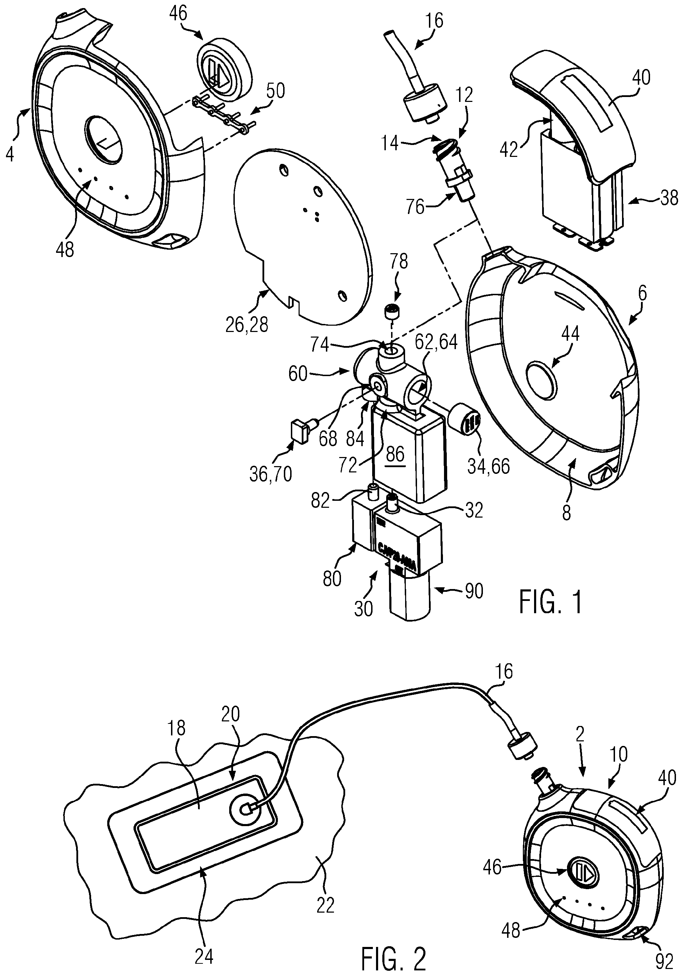

Figure 1 exemplifies a pumping device 2 with a first housing shell element 4 and a second housing shell element 6, which are sealingly connected with each other for defining a housing compartment 8. As evident in particular from figures 2 to 5, a housing 10 surrounding said housing compartment 8 is formed by the two shell elements 4, 6 and is outwardly projected by a pressure interface body 12, which defines a pressure interface 14 accessible from outside for being connected to a fluid connection.

This fluid connection in figure 2 is identified with reference numeral 16 and provided by a hose leading to a dressing 18, which dressing comprises a pressure tight covering 20 adhered to skin 22 of a user surrounding a wound 24.

Turning back to figure 1, the housing compartment 8 contains a PCB 26 forming part of a controller 28. The controller 28 is operatively coupled to a pump 30 for controlling operation of the pump 30. The pump 30 comprises a suction port 32 making the suction of the pump 30 availabe.

Reference number 34 identifies a pressure relief valve. Reference number 36 identifies a pressure sensor, which pressure sensor 36 is operatively coupled to the controller 28 and provided on the PCB 26 as a surface mounted device. Within the housing compartment 8, there is provided a battery compartment 38, which is open to an outer circumference of the housing 10 and covered by a battery compartment cover 40. This battery compartment cover 40 sealingly cooperates with the two housing shell elements 4, 6 to sealingly enclose the battery compartment 38, which battery compartment 38 is adapted to receive two AAA batteries for supplying the pump device 2 with energy and in particular energize the pump 30. In order to be a small battery compartment cover 40 the same as arranged at the axial end of the AAA battery i.e. opposite to the +/- pole of the battery.

A main side of the second housing shell element 6 is provided with holes being covered by a hydrophobic filter 44, which is permeable to gas/air and not permeable to water and thus avoids water from entering into the housing compartment 8. The opposite main side surface of the housing 10 provided by the first housing shell element 4 is provided with an on/off switch 46 and provided with translucent sections 48 covering an LED arrangement 50, which as the switch 46 is surface mounted on the PCB 26.

Reference number 60 identifies a manifold body of an elastic plastic material, which surrounds a manifold chamber 62 in communication with a plug connection receptacle 64 for a pressure relief body 66 of the pressure relief valve 34. The manifold body 60 furthermore provides a plug connection receptacle 68 for a pressure sensor body 70 of the pressure sensor 36, a plug connection receptacle 72 for the suction port 32 and a plug connection receptacle 74 for a male connection section 76 of the pressure interface body 12. Between set plug connection receptacle 76 and the plug connection receptacle 72 for the suction port 32, the manifold body 60 receives and sealingly holds a check valve 78, which prevents backflow from the pump 30 into the manifold chamber 62 after the pump has terminated the application of suction, which backflow may reduce the suction pressure within the manifold chamber 62. The check valve 78 can be plug connected in a sealing way inside the manifold chamber 62 in particular on the side of the suction port, but will preferably be inserted into the manifold body 60 through the plug connection receptacle 74 32. The manifold body 60 may form a valve seat which prevents dislocation of the check valve 78 towards the suction port. The valve seat may define a shoulder against which the check valve 78 rests. Alternatively, the check valve 78 is arranged on the side of the pressure interface body 12.

Reference number 80 identifies a vent valve, which may operatively be coupled with the controller 28 and thus connected with the PCB 26 for venting the manifold chamber 62 and thus reducing the pressure within the dressing 18. Respective vent valve 80 has a vent valve port 82, which is sealingly received within a plug connection receptacle 84 of the manifold body 60. In case the vent valve 80 is omitted for cost or weight reasons, the vent valve plug connection receptacle 84 may be blocked by a plastic plug. The control of the pump may order activation of the vent valve 30 to relieve the wound from the negative suction pressure. Alternatively, the control may comprise information to stop suction performance and thereby allow to reduce the suction pressure within the wound due to the inherent leakage.

Each of the aforementioned plug connection receptacles sealingly enclose and receive respective bodies and/or ports. In other words, the manifold body 60 is the central component within the housing compartment 8 that fluidically connects each and every component effecting, controlling and measuring the suction pressure generated by the pumping device 2.

Due to the elastic properties of the manifold body 60, each of the components is arranged within the housing compartment 8 in a soundproofing manner.

The manifold body 60 furthermore provides a sleeve 86, which at least in part receives and holds the pump 30, thereby supporting respective pump 30 in a soundproofing manner within the housing. The pump 30 has a pump housing 90, which is partially or fully covered by the sleeve 86, but allows air to vent out of the pump 30. The pump 30 has no extra venting port. Thus, the noise emission of the pump 30 is very low with less than 40 dBA, preferably less than 35 dBA.

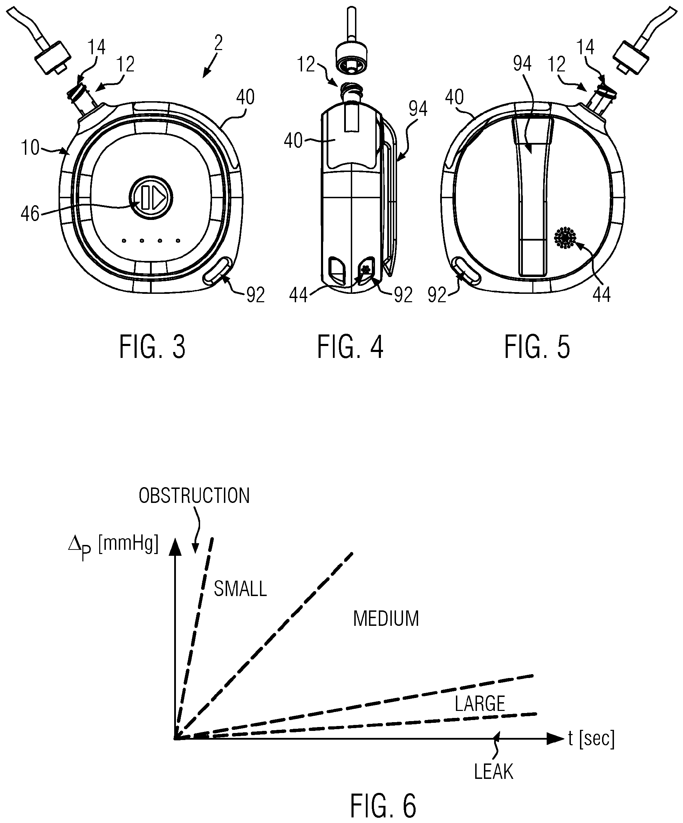

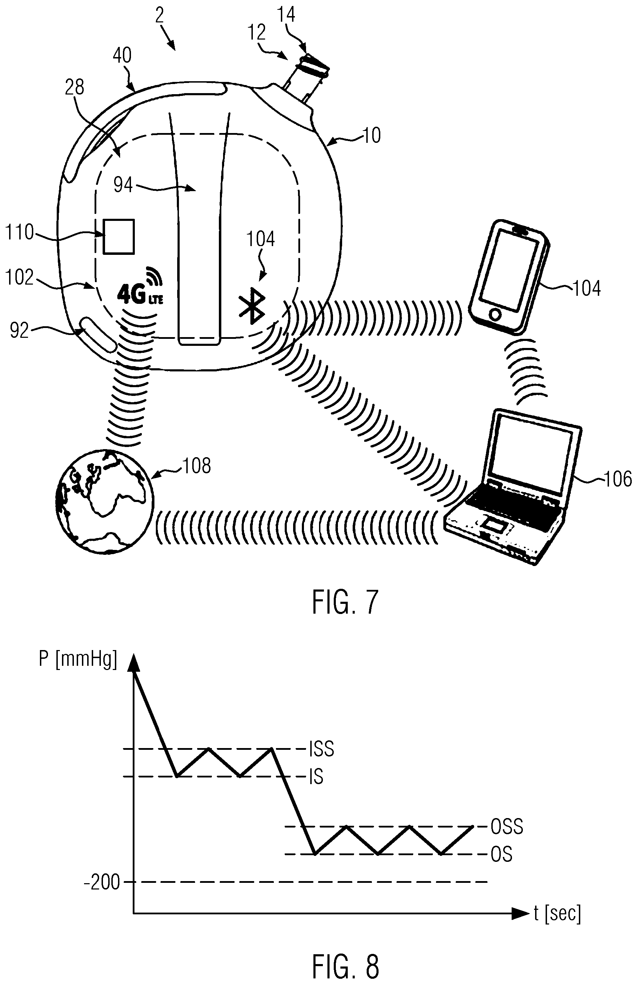

In the following, means for holding or suspending the pumping device 2 are discussed. In particular, figures 3 through 5 show an eyelet 92, which allows a carrying strap (not shown) to be guided there through for hanging the pumping device 2 on a hook or the like. Figure 4 exemplifies a variant position of the hydrophobic filter 44 underneath said eyelet 92. For fastening the pumping device 2 on a belt or a waistband, the housing 10 is provided with an integrated fastening clip 94. In the variant of figure 7, the fastening clip 94 is slimmer.

Figure 6 exemplifies the capability of the controller 28 to select an appropriate suction yield of the pump depending on the size of the dressing 18. In case of a small dressing 18 an initial suction pressure provided to evacuate the dressing 18 will result in a rather steep gradient of the pressure difference versus time. In case the dressing 18 is rather large respective gradient is slow. Both gradients will thus be evaluated by the controller 28 based on the measurements of the pressure sensor 36 to appropriately set the suction yield. In case of a small dressing 18 the suction yield will be selected by the controller 28 lower than in case of a large dressing 18 in which the suction yield of the pump 30 will be selected higher. This feature leads to an economical operation of the pump 30 and thus the lowest possible energy consumption for operating the pump 30.

Furthermore and as exemplified in figure 6 a very steep gradient results in a conclusion that the vacuum path between the pump 30 and the dressing 18 is obstructed. The respective pressure gradient will thus result in release of an acoustic or haptic or visual warning issued by the controller 28 which may be noticeable by the user of the pumping device 2 by vibration or sound and/or light emitted from the pumping device 2. A respective warning will be released in case of leak, which leak will be detected in case a very low pressure gradient is detected by the pressure sensor 36. Obviously, the quality of the alarm released in case of obstruction shall be different from the alarm in case of leak in order to allow discrimination between the two incidences leading to the alarm.

Figure 7 shows various interfaces provided with the pumping device 2, specifically in form of interfaces provided on the PCB 30 of the controller 28 drawn in figure 7 in hashed lines. The pumping device 2 may have a Bluetooth interface 100 and/or an LTE interface 102 which are operatively connected with a mobile device 104 zum www connected, a PC 106 and/or the World Wide Web 108. The mobile device 104 and the PC 104 for example can likewise be wirelessly connected with each other. The PC 106 and/or the mobile device 104 may form a docking station 112 for reading in and reading out data stored on a memory 110 provided on the PCB 30. This memory of 110 can inter alia comprise information on the total running time of the pumping device 2 which may be over read via the docking station 112. Moreover the memory 110 may store operational parameters or information sensed by e.g., the pressure sensor 36.

The memory 110 may likewise be adapted to store an initial suction level, which is identified with reference numeral IS in figure 8 which identifies the signal of the pressure signal over time at time equal zero. The dressing 18 is primed and thus placed under suction pressure for the first time. The initial suction level IS has a higher absolute pressure than an operational suction level OS and is ordered by the controller 28 to acquaint the body to the negative pressure on the ground of the wound 24. When reaching the initial suction level IS operation of the pump 30 is automatically stopped which due to a certain degree of leakage will eventually lead to a rise of the pressure within the dressing 18. ISS identifies an upper threshold value for the initial suction level IS, which will cause the controller 28 to start operation of the pump 30 until the initial suction level IS is sensed by the pressure sensor 36. After a certain period of time, which may be a predetermined value stored in the memory 110 or be entered for storage by clinical personnel into the memory 110, the controller 28 orders the operational suction level OS, which will then usually be maintained until the end of the negative pressure therapy. OSS identifies an upper threshold value for the operational suction level OS, which will cause the controller 28 to start operation of the pump 30 until the operational suction level OS is sensed by the pressure sensor 36.

The lowest horizontal dotted line in figure 8 identifies the lowermost pressure attainable by the pumping device. At a suction pressure below this value (here - 200 mmHg), the pressure relief valve 34 will automatically open to avoid undue low suction effective in the wound 24.
1. The present description in particular relates to the following subject matters and inventions:A pumping device (2) for reduced pressure therapy of a wound (24) covered by a dressing (18), the pumping device (2) comprising a housing (10), a pump (30) comprising a suction port (32), a controller (28) configured to selectively drive the pump, a battery compartment (38) adapted to receive at least one battery (42) for energizing the pump, a pressure interface (14) in fluid communication with said suction port (32), which pressure interface (14) is accessible from outside for being connected to a fluid connection (16) leading to the dressing (18), at least one of a pressure relief valve (34) and a pressure sensor (36) adapted to provide a signal indicative of a suction pressure at the suction port (32) **characterized by** a manifold body (60) for fluidically connecting the suction port (32), the pressure interface (14) and the at least one of said pressure relief valve (34) and said pressure sensor (36).
2. The pumping device (2) as defined in claim 1, wherein said manifold body (60) provides plug receptacles for plug connecting the suction port (32), the pressure interface (14) and the at least one of said pressure relief valve (34) and said pressure sensor (36).
3. The pumping device (2) as defined in claim 1 or 2, wherein said manifold body (60) is made of an elastic material for sealingly surrounding the suction port (32), a pressure interface body (12) providing the pressure interface (14) and the at least one of a pressure relief valve body of said pressure relief valve (34) and a pressure sensor body (70) of said pressure sensor (36).
4. The pumping device (2) as defined in any of the preceding claims, wherein said manifold body (60) provides at least five plug receptacles.
5. The pumping device (2) as defined in any of the preceding claims, wherein said manifold body (60) is provided with a sleeve (86) adapted to at least in part receive the pump (30).
6. The pumping device as defined in any of the preceding claims, wherein said pump (30) is adapted to vent by leaking air through a pump housing (90) of the pump.
7. A pumping device (2) for reduced pressure therapy of a wound (24) covered by a dressing (18), the pumping device (2) comprising a housing (10), a pump (30) comprising a suction port (32), a controller (28) configured to selectively drive the pump, a battery compartment (38) adapted to receive at least one battery (42) for energizing the pump, a pressure interface (14) in fluid communication with said suction port (32), which pressure interface (14) is accessible from outside for being connected to a fluid connection (16) leading to the dressing (18), at least one of a pressure relief valve (34) and a pressure sensor (36) adapted to provide a signal indicative of a suction pressure at the suction port (32), preferably the pumping device (2) as defined in any of the preceding claims, wherein said housing (10) is formed of at least two housing shell elements (4,6), which housing shell elements (4,6) are sealingly connected with each other to hermetically seal a housing compartment (8), which housing compartment (8) is in communication with ambient only via a hydrophobic filter (44), which is permeable to gas and not permeable to water.
8. The pumping device (2) as defined in claim 9, wherein said at least two housing shell elements sealingly sandwich there between a pressure interface body (12) providing the pressure interface (14).
9. The pumping device (2) as defined in any of the preceding claims, wherein an eyelet (92) is provided on an outer side of said housing (10) and wherein a carrying strap is guided through said eyelet (92).
10. The pumping device (2) as defined in preceding claims 7 and 9, wherein said hydrophobic filter (44) is provided underneath the carrying strap.
11. The pumping device (2) as defined in any of the preceding claims, wherein said housing (10) is provided with an integrated fastening clip (94).
12. A pumping device (2) for reduced pressure therapy of a wound (24) covered by a dressing (18), the pumping device (2) comprising a housing (10), a pump (30) comprising a suction port (32), a controller (28) configured to selectively drive the pump, a battery compartment (38) adapted to receive at least one battery (42) for energizing the pump, a pressure interface (14) in fluid communication with said suction port (32), which pressure interface (14) is accessible from outside for being connected to a fluid connection (16) leading to the dressing (18), at least one of a pressure relief valve (34) and a pressure sensor (36) adapted to provide a signal indicative of a suction pressure at the suction port (32), preferably the pumping device (2) as defined in any of the preceding claims, wherein said housing (10) provides a battery compartment (38) adapted to receive one or two AAA type batteries (42).
13. The pumping device (2) as defined in claim 12, wherein said housing (10) provides a battery compartment (38) adapted to receive at least one AAA type battery (42), which battery compartment (38) is releasably and sealingly covered by a battery compartment cover (40) provided above the battery compartment (38) at the longitudinal end of the AAA type battery.
14. A pumping device (2) for reduced pressure therapy of a wound (24) covered by a dressing (18), the pumping device (2) comprising a housing (10), a pump (30) comprising a suction port (32), a controller (28) configured to selectively drive the pump, a battery compartment (38) adapted to receive at least one battery (42) for energizing the pump, a pressure interface (14) in fluid communication with said suction port (32), which pressure interface (14) is accessible from outside for being connected to a fluid connection (16) leading to the dressing (18), at least one of a pressure relief valve (34) and a pressure sensor (36) adapted to provide a signal indicative of a suction pressure at the suction port (32), preferably the pumping device (2) as defined in any of the preceding claims, wherein said housing (10) provides a battery compartment (38) sealingly and releasably covered by a battery compartment cover (40) secured to the housing (10) by means of a force-locked and/or a positive connection.
15. The pumping device (2) as defined in any of the preceding claims, wherein said housing (10) provides a battery compartment (38) releasably covered by a battery compartment cover (40) connected to the housing (10) by a snap connection, which snap connection sealingly presses the battery compartment lid against the housing (10).
16. A pumping device (2) for reduced pressure therapy of a wound (24) covered by a dressing (18), the pumping device (2) comprising a housing (10), a pump (30) comprising a suction port (32), a controller (28) configured to selectively drive the pump, a battery compartment (38) adapted to receive at least one battery (42) for energizing the pump, a pressure interface (14) in fluid communication with said suction port (32), which pressure interface (14) is accessible from outside for being connected to a fluid connection (16) leading to the dressing (18), at least one of a pressure relief valve (34) and a pressure sensor (36) adapted to provide a signal indicative of a suction pressure at the suction port (32), preferably the pumping device (2) as defined in any of the preceding claims, wherein the controller (28) is operatively connected with said pressure sensor (36) and configured to evaluate a pressure gradient calculated from pressure values sensed by said pressure sensor (36) the during suction priming the dressing (18) for selectively setting a suction yield of said pump (30).
17. The pumping device (2) as defined in any of the preceding claims, wherein the controller (28) is adapted to apply an initial suction level (IS) and order an operational suction level (OS) after a predetermined running time of the pumping device (2), wherein the initial suction level (IS) has a higher minimum pressure than the operational suction level (OS).
18. The pumping device (2) as defined in any of the preceding claims, wherein the controller (28) is operatively connected with said pressure sensor (36) and configured to evaluate a pressure gradient calculated from pressure values sensed when setting or holding the dressing (18) under a negative pressure for identifying a leak.
19. The pumping device (2) as defined in any of the preceding claims, wherein the controller (28) is operatively connected with said pressure sensor (36) and configured to evaluate a pressure gradient calculated from pressure values sensed when setting or holding the dressing (18) under a negative pressure for identifying an obstruction.
20. The pumping device (2) as defined in claim 18 or 19, wherein the controller (28) is configured to release an audible and/or haptic and/or visual alarm in case of a leak and/or an obstruction is identified.
21. The pumping device (2) as defined in any of the preceding claims, wherein the controller (28) comprises a memory (110) configured to store data including operational parameters of said pumping device (2) and signals sensed by at least one sensor of said pumping device (2) and further comprising an interface for reading out said stored data.
22. The pumping device (2) as defined in claim 21, further comprising a docking station which can be brought in data connection with the interface for reading out stored data from the memory (110) or reading in data into the memory (110).
23. A pumping device (2) for reduced pressure therapy of a wound (24) covered by a dressing (18), the pumping device (2) comprising a housing (10), a pump (30) comprising a suction port (32), a controller (28) configured to selectively drive the pump, a battery compartment (38) adapted to receive at least one battery (42) for energizing the pump, a pressure interface (14) in fluid communication with said suction port (32), which pressure interface (14) is accessible from outside for being connected to a fluid connection (16) leading to the dressing (18), at least one of a pressure relief valve (34) and a pressure sensor (36) adapted to provide a signal indicative of a suction pressure at the suction port (32), preferably the pumping device (2) as defined in any of the preceding claims, further comprising an acceleration sensor for sensing the movement of the pumping device (2), wherein the acceleration sensor is operatively connected to said controller (28) for controlling operation of the pumping device in response to a sensed movement of the pumping device (2).
24. The pumping device (2) as defined in any of the preceding claims, further comprising an acceleration sensor for sensing the movement of the pumping device (2), wherein the acceleration sensor is operatively connected to said memory (110) to store movement data indicative of the movement of the pumping device.
25. The pumping device (2) as defined in any of the preceding claims, wherein the pumping device (2) comprises a single switch (46), which switch (46) is provided on the outer side of the housing (10) for activating the pumping device (2).
26. A pumping device (2) for reduced pressure therapy of a wound (24) covered by a dressing (18), the pumping device (2) comprising a housing (10), a pump (30) comprising a suction port (32), a controller (28) configured to selectively drive the pump, a battery compartment (38) adapted to receive at least one battery (42) for energizing the pump, a pressure interface (14) in fluid communication with said suction port (32), which pressure interface (14) is accessible from outside for being connected to a fluid connection (16) leading to the dressing (18), at least one of a pressure relief valve (34) and a pressure sensor (36) adapted to provide a signal indicative of a suction pressure at the suction port (32), preferably the pumping device (2) as defined in any of the preceding claims, wherein the controller (28) of the pumping device (2) is combinable with a computer application run on a mobile device (104) for visualizing and/or adjusting at least one operational parameter of the pumping device (2).
27. A computerized method for reduced pressure therapy management comprising:
   inputting via an input interface input information related to the operation of a pumping device (2) for the reduced pressure therapy of a wound (24) covered by a dressing (18);
   outputting via an output interface an indication to the user;
   processing via a processing circuitry preferably configured by an app the input information obtained via the input interface to obtain an indication on at least one operational parameter and outputting via the output interface the indication to the user.
28. A computerized method for reduced pressure therapy management, preferably according to claim 27 comprising:
   inputting via the input interface input information related to the operation of a pumping device (2) for the reduced pressure therapy of a wound (24) covered by a dressing (18);
   outputting via an output interface an indication to the user;
   processing via a processing circuitry preferably configured by an app input information obtained via the input interface and pre-stored calendar information, to obtain an operational parameter to pause operation of the pumping device (2), to transmit via a pump interface the operational parameter to the pumping device (2), and to provide an indication on pausing the operation of the pumping and outputting via the output interface the indication to the user.
29. The computerized method for reduced pressure therapy management according to claim 27 or 28, further comprising: processing via a processing circuitry configured by the app input healing progress information optionally input sensor information of at least one sensor of the pumping device (2), correlate healing progress information by Al with input information related to the operation of a pumping device (2) and/or the input sensor information, output an Al generated evaluation of pumping operation parameters or sensor information versus healing process via the output interface.
30. The computerized method for reduced pressure therapy management according to any of the claims 27 to 29, further comprising:
   processing via the processing circuitry configured by the app a help request entered by the user via the input interface,
   correlate via the processing circuitry preferably configured by the app prestored instruction for use with the help request to obtain help information and outputting via the output interface the help information to the user.
31. A computer application adapted to, when run on a mobile device (104) or PC (106) or laptop or cloud perform the computerized method for reduced pressure therapy management according to any of the claims 27 to 30.
32. A negative pressure therapy kit, comprising the pumping device (2) of any of the claims 1 to 28, a dressing (18), a fluid connection (16) coupleable with the dressing (18) and the pumping device (2) and configured to provide a fluid path of reduced pressure between the dressing (18) and the pumping device (2), wherein the negative pressure kit is sterile and contained in sterile condition in at least one packaging element.

### List of References

- 2: Pumping device
- 4: First housing shell element
- 6: Second housing shell element
- 8: Housing compartment
- 10: Housing
- 12: Pressure interface body
- 14: Pressure interface
- 16: Fluid connection
- 18: Dressing
- 20: Pressure tight covering
- 22: Skin
- 24: Wound
- 26: PCB
- 28: Controller
- 30: Pump
- 32: Suction port
- 34: Pressure relief valve
- 36: Pressure sensor
- 38: Battery compartment
- 40: Battery compartment cover
- 42: Battery
- 44: Hydrophobic filter
- 46: Switch
- 48: Translucent sections
- 50: LED arrangement
- 60: Manifold body
- 62: Manifold chamber
- 64: Plug connection receptacle for the pressure relief valve 34
- 66: Pressure relief body
- 68: Plug connection receptacle for the pressure sensor 36
- 70: Pressure sensor body
- 72: Plug connection receptacle for the suction port 32
- 74: Plug connection receptacle for the pressure interface body 12
- 76: Male connection section
- 78: Check valve
- 80: Vent valve
- 82: Vent valve port
- 84: Plug connection receptacle for the vent valve 80
- 86: Sleeve
- 90: Pump housing
- 92: Eyelet
- 94: Integrated fastening clip
- 100: Bluetooth interface
- 102: LTE interface
- 104: Mobile device
- 106: PC
- 108: World Wide Web
- 110: Memory
- 112: Docking station
- IS: Initial suction level
- OS: Operational suction level
- ISS: Threshold value for the initial suction level
- OSS: Threshold value for the operational suction level

## Claims

1. A pumping device (2) for reduced pressure therapy of a wound (24) covered by a dressing (18), the pumping device (2) comprising a housing (10), a pump (30) comprising a suction port (32), a controller (28) configured to selectively drive the pump, a battery compartment (38) adapted to receive at least one battery (42) for energizing the pump, a pressure interface (14) in fluid communication with said suction port (32), which pressure interface (14) is accessible from outside for being connected to a fluid connection (16) leading to the dressing (18), at least one of a pressure relief valve (34) and a pressure sensor (36) adapted to provide a signal indicative of a suction pressure at the suction port (32) **characterized by** a manifold body (60) for fluidically connecting the suction port (32), the pressure interface (14) and the at least one of said pressure relief valve (34) and said pressure sensor (36).

2. The pumping device (2) as defined in claim 1, wherein said manifold body (60) provides plug receptacles for plug connecting the suction port (32), the pressure interface (14) and the at least one of said pressure relief valve (34) and said pressure sensor (36).

3. The pumping device (2) as defined in claim 1 or 2, wherein said manifold body (60) is made of an elastic material for sealingly surrounding the suction port (32), a pressure interface body (12) providing the pressure interface (14) and the at least one of a pressure relief valve body of said pressure relief valve (34) and a pressure sensor body (70) of said pressure sensor (36).

4. The pumping device (2) as defined in any of the preceding claims, wherein said manifold body (60) provides at least five plug receptacles.

5. The pumping device (2) as defined in any of the preceding claims, wherein said manifold body (60) is provided with a sleeve (86) adapted to at least in part receive the pump (30).

6. The pumping device as defined in any of the preceding claims, wherein said pump (30) is adapted to vent by leaking air through a pump housing (90) of the pump.

7. A pumping device (2) as defined in any of the preceding claims, wherein the controller (28) is operatively connected with said pressure sensor (36) and configured to evaluate a pressure gradient calculated from pressure values sensed by said pressure sensor (36) the during suction priming the dressing (18) for selectively setting a suction yield of said pump (30).

8. The pumping device (2) as defined in any of the preceding claims, wherein the controller (28) is adapted to apply an initial suction level (IS) and order an operational suction level (OS) after a predetermined running time of the pumping device (2), wherein the initial suction level (IS) has a higher minimum pressure than the operational suction level (OS).

9. The pumping device (2) as defined in any of the preceding claims, wherein the controller (28) is operatively connected with said pressure sensor (36) and configured to evaluate a pressure gradient calculated from pressure values sensed when setting or holding the dressing (18) under a negative pressure for identifying a leak.

10. The pumping device (2) as defined in any of the preceding claims, wherein the controller (28) is operatively connected with said pressure sensor (36) and configured to evaluate a pressure gradient calculated from pressure values sensed when setting or holding the dressing (18) under a negative pressure for identifying an obstruction.

11. The pumping device (2) as defined in claim 9 or 10, wherein the controller (28) is configured to release an audible and/or haptic and/or visual alarm in case of a leak and/or an obstruction is identified.

12. The pumping device (2) as defined in any of the preceding claims, wherein the controller (28) comprises a memory (110) configured to store data including operational parameters of said pumping device (2) and signals sensed by at least one sensor of said pumping device (2) and further comprising an interface for reading out said stored data.

13. The pumping device (2) as defined in claim 12, further comprising a docking station which can be brought in data connection with the interface for reading out stored data from the memory (110) or reading in data into the memory (110).

14. A pumping device (2) as defined in any of the preceding claims, further comprising an acceleration sensor for sensing the movement of the pumping device (2), wherein the acceleration sensor is operatively connected to said controller (28) for controlling operation of the pumping device in response to a sensed movement of the pumping device (2).

15. The pumping device (2) as defined in any of the preceding claims, further comprising an acceleration sensor for sensing the movement of the pumping device (2), wherein the acceleration sensor is operatively connected to said memory (110) to store movement data indicative of the movement of the pumping device.
